# EUROPEAN PATENT APPLICATION

(11) **EP 2 070 906 A1**
(43) Date of publication of application: **17.06.2009**
(21) Application number: 07398018.7
(22) Date of filing: 06.12.2007
(51) Int. Cl.: C07C 49/417, C07J 63/00

(54) **Extraction and purification of friedelin**

(71) Applicant: Amorim Revestimentos, S.A., 4536-907 S. Paio de Oleiros (PT)
(72) Inventor: Pires, Ricardo Alexandre Rodrigues, 1500-548 Lisboa (PT); Martins, Susana Pinto Araújo da Silva Estima, 4470-193 Maia (PT); Chagas, José António Marchão Das, 4500-669 Silvalde (PT); Reis, Rui Luís Gonçalves Dos, 4250-242 Porto (PT)
(74) Representative: Alves Moreira, Pedro

(57) **Abstract**

Friedelin is a natural compound with promising proprieties. On its own or with chemical modification it is possible to introduce relevant biological activities, e.g. anti-cancer, anti-aging and agrochemical. Its availability in significant amounts has been a major drawback on its regular use and in the pursuit of different applications. Cork and cork-derived materials (e.g. black condensate) are the most relevant sources of Friedelin in nature (up to 10% in concentration). The present invention reports straightforward procedures to extract and purify Friedelin from cork and cork-derived materials (e.g. black condensate). It uses solvent extraction and (re)crystallization techniques easy to scale up to an industrial level, with a low solvent and low time consumption, high yields, up to 2.9%, and high purity degrees, up to 96%.

## Description

### FIELD OF THE INVENTION

Friedelin is one of the most abundant terpenes present in cork and cork-derived materials. Recent studies showed that Friedelin and, particularly, its derivates have anti-cancer activity (Moiteiro et al., J. Nat. Prod., 64 (2001), 1273-1277; Moiteiro et al., J. Nat. Prod., 67 (2004), 1193-1196; Moiteiro et al., W009026, 1994; Zhang et al., WO052383A1, 2004), analgesic and anti-inflammatory capability (Nakamura et al., EP0776666A2, 1997), anti-bacterial activity (Frame, A.D., WO059371A1, 2003), vascularizing agent (Mitsuhiro et al., JP068972, 2002), have potential to be used in pharmaceuticals or functional foods for the treatment or prevention of cardiovascular and cerebrovascular diseases and tumours (Zhang et al., US0148733, 2006), potential to be used in cosmetics (Kinshi et al., JP012509, 2003) and as agrochemical (Moiteiro et al., J. Agric. Food Chem., 54 (2006), 3566-3571). Its anti-oxidizing capacity is under study.

The availability of Friedelin in large quantities has been a major drawback in the development of applications with industrial relevance. The procedures described in this invention allow the extraction of Friedelin from cork and cork-derived materials, such as cork extracts, cork powder, granules, planks, but in particular from one of the cork industry by-products: black condensate.

Black condensate is usually produced in large quantities by the insulation corkboard industry. This by-product has no relevant application and is usually incinerated. This invention gives an added value to this by-product as a source of fine and speciality chemicals (e.g. Friedelin).

### SUMMARY OF THE INVENTION

The present invention describes a straightforward procedure of extraction and purification of Friedelin from cork and cork-derived materials by means of alkaline hydrolysis, solvent extraction/washing and (re)crystallization techniques using low amounts of isolated or mixtures of solvents. With the present invention, it is possible to obtain Friedelin with a purity degree of up to 96% and with an overall extraction yields of up to 2.9% (corresponding to, approximately, 30% to 50% of the amount of Friedelin present in the raw material)

### BACKGROUND OF THE INVENTION

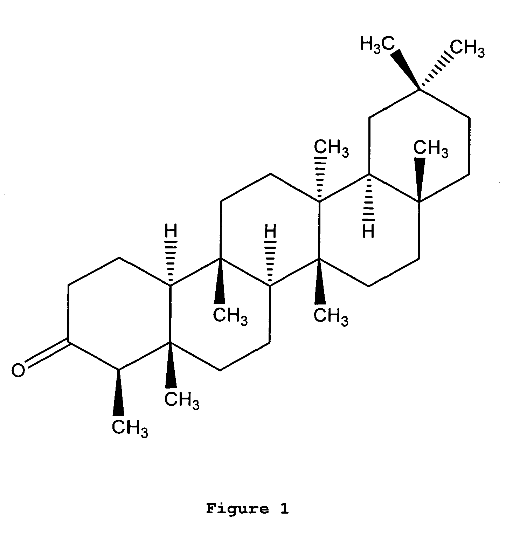

Cork is one of the most relevant sources of Friedelin in nature. Natural and treated cork, in powder or granules, is described to have a significant amount of Friedelin (chemical structure presented in Figure 1). Additionally, during insulation corkboard production a by-product called black condensate is formed. Having no relevant application is usually incinerated, although, it is a Friedelin-rich material (1-10% in weight concentration) (Sousa et al., J. Agric. Food Chem., 54 (2007), 6888-6893).

Procedures of isolating Friedelin from black condensate have been described (Júnior et al., PT101683A, 1996; Júnior et al., EP1103539A1, 2001) using organic solvent fractionation and extraction with aliphatic hydrocarbons, although, the described methodologies require multi-step procedures that include 5 to 11 steps to reach low purity Friedelin (40% to 60%) and 10 to 12 steps to obtain a sample of high purity Friedelin (≥95%) . Additionally, considering the overall quantity of solvents used, the methodologies described in the literature consume between 0.92L and 1.18L of solvents per gram of high purity Friedelin (≥95%).

More recently, extraction and purification of terpenoids (including Friedelin) from *Heritiera littoralis* D. has been described using chromatographic fractionation technology (Tian et al., CN1580071, 2005). All the reported cases, describe procedures that are time and solvent consuming.

In the case of Friedelin and its derivatives, it has been reported several interesting biological activities (e.g. Moiteiro et al., W009026, 1994). The anti-cancer potential of a Friedelin derivative (2,3-secofriedelan-2-al-3-oic acid) has been reported to have an inhibitory effect on the growth of breast, renal, melanoma, lung and central nervous system cancer cells (Moiteiro et al., J. Nat. Prod., 64 (2001), 1273-1277). Additionally, in the case of 3-nor-2,4-secofriedelan-4-oxo-2-oic acid (another Friedelin derivative) it was also reported its inhibitory action in the growth of breast, lung and central nervous system cancer cells (Moiteiro et al., J. Nat. Prod., 67 (2004), 1193-1196). More recently, Friedelin and its derivatives have been studied as agrochemicals for crop protection. The study showed promising results for several Friedelin derivatives against *S*. *littoralis* and *L. decemlineata* (Moiteiro et al., J. Agric. Food Chem., 54 (2006), 3566-3571).

The increasing number of possible applications for Friedelin and its derivatives demands for sources where it can be found in high concentrations. On the other hand, the development of Friedelin extraction, isolation and purification procedures with an industrially appealing yield, cost and purity, are needed. This invention pretends to give an answer to this demand, reporting a simple and straightforward solvent extraction procedure to obtain Friedelin from cork and cork-derived materials.

### DETAILED DESCRIPTION OF THE INVENTION

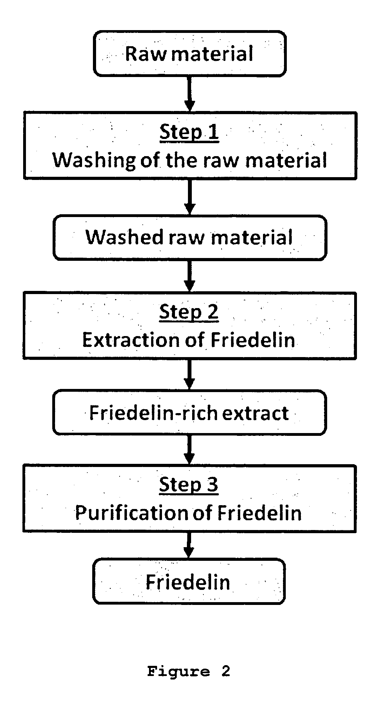

The present invention consists of a three to four steps procedure with a low time and low solvent consumption. The first step is to obtain a material with a reduced concentration of contaminants, from which is possible to extract Friedelin. The second step focuses on the extraction of Friedelin, while in the third step a purification of the extracted Friedelin is carried out. A scheme of the procedure is presented in Figure 2.

At the first step a treatment (washing) of the raw material (e.g. black condensate) with an aliphatic alcohol for time periods up to 24h and from room to the boiling temperature of the solvent is performed, preferably at the boiling temperature. The use of an alkaline alcoholic solution is preferred. At this step a Soxhlet experimental apparatus can also be used. Optionally, an additional washing of the filtration residue with an aliphatic alcohol can improve the purity of the final extract (obtained in the next step).

In the second step Friedelin is extracted from the washed raw material using solvent extraction techniques. In this step, an apolar solvent is preferably used (e.g. chloroform, methylene chloride, etc.), from room to reflux temperatures and from time periods up to 24h. The evaporation of the solvent yields a Friedelin-rich solid extract.

The third step is the purification of the Friedelin-rich extract by (re)crystallization technique using halogenated solvents, aliphatic hydrocarbons, aliphatic alcohols, ketones, ethers, esters, carboxylic acids and/or acetates as isolated solvents or mixtures (e.g. n-hexane, n-hexane:ethyl acetate), with or without the addition of activated charcoal. This (re)crystallization step can be repeated to obtain higher purity samples.

An alternative or sequential technique (optional fourth step) is to solubilise the Friedelin-rich material in one of the previously described solvents (isolated or in mixtures) with increased temperature and with the addition of activated charcoal. Higher purity Friedelin can be obtained after filtration of the solution and evaporation of the solvent. If necessary, a final wash of the Friedelin sample with an aliphatic alcohol (e.g. ethanol) might be necessary to separate contaminant remains.

The described examples are based on black condensate due to its complex matrix and the higher probability of having contaminations during the purification steps. The described procedures can be executed with a lower consumption of solvent than previous patents. With the present straightforward four steps procedures it is possible to spend less than 0.55L of solvent to obtain one gram of high purity Friedelin (≥95% purity). Additionally, taking the previously described into consideration, a successful procedure to extract Friedelin from black condensate can also be applied in less complex matrixes as, for example, most of the solvent extracts of cork and cork-derived materials.

### Example 1

Black condensate powder (m=204.9g) was extracted with 2L of 2% sodium hydroxide ethanolic solution during 6 hours under reflux. The mixture was cooled down to room temperature and filtrated under vacuum. The filtration residue was dried yielding 116.8g of raw material for Friedelin extraction.

Chloroform (V=1170mL) was added to the material obtained from the previous step (m=116.8g) and stirred during 30min at room temperature. The mixture was filtrated under vacuum and the solvent (that might be reused) was removed from the extract and collected using a rotator evaporator. The residual chloroform was evaporated at room temperature, yielding 18.5g of a Friedelin-rich extract, corresponding to an extraction yield of 9.0%.

The Friedelin-rich extract (m=15.0g) was dissolved in hot n-hexane:ethyl acetate system in the presence of activated charcoal. After filtration of the charcoal (re)crystallization yielded 8.1g of Friedelin, corresponding to an overall extraction yield of 4.9%.

Higher purity Friedelin was obtained dissolving 1.0g of Friedelin from the previous step in n-hexane hot and adding activated charcoal to the hot solution, under constant heating and stirring, for 30min. The charcoal was filtered and the solvent was evaporated yielding 0.6g of Friedelin.

The procedure described in this example produced Friedelin with a purity degree of 96% and an overall yield of extraction of 2.9%. This example is a four step procedure that uses ~0.53L of solvent per gram of high purity Friedelin.

### Example 2

Black condensate powder (m=204.3g) was extracted with 2L of 3% potassium hydroxide methanolic solution during 3h under reflux. The mixture was cooled down to room temperature and filtered under vacuum. The filtration residue was dried yielding 148.7g of raw material for Friedelin extraction.

Chloroform (V=1490mL) was added to the material obtained from the previous step (m=148.7g), stirred during 30min at room temperature. The mixture was filtrated under vacuum and the solvent (that might be reused) was removed from the extract and collected using a rotator evaporator. The residual chloroform was evaporated at room temperature, yielding 11.8g of a Friedelin-rich extract, corresponding to an extraction yield of 5.8%.

The Friedelin-rich extract (m=10.0g) was dissolved in hot n-hexane:ethyl acetate system in the presence of activated charcoal. After filtration of the charcoal (re)crystallization yielded 4.3g of Friedelin, corresponding to an overall extraction yield of 2.5%.

The Friedelin obtained from the previous step (m=1.0g) was dissolved in n-hexane hot. Activated charcoal was added to this solution, under constant heating and stirring, for 30min. The charcoal was filtered and the solvent was evaporated yielding 0.5g of Friedelin.

The procedure described in this example produced Friedelin with a purity degree of 61% and an overall yield of extraction of 1.2%.

## Claims

1. A three to four step procedure, with a low time and low solvent consumption, to extract Friedelin from cork and cork-derived materials, wherein the first step is a washing of the raw material with an alkaline alcoholic solution producing a residue from which, in the second step, Friedelin is extracted using apolar solvents, whose extract, in the third step, Friedelin is (re)crystallized, with or without the addition of activated charcoal, using isolated solvent or mixtures and, in the optional fourth step, Friedelin is purified with an apolar solvent hot in the presence of activated charcoal.

2. Solvent extraction procedure as described in claim 1, where the described raw materials can be black condensate, cork extracts, cork powder, granules or planks.

3. Procedure as described in claim 1, where in the first step the alkalis might be calcium carbonate, magnesium hydroxide or, preferably, potassium or sodium hydroxide.

4. Procedure as described in claim 1, wherein the first step the alcohols might be aliphatic, aromatic or cyclic, primary, secondary or tertiary alcohols such as methanol, ethanol, 1-propanol, 1-butanol, 2-butanol, isopropanol, preferably methanol, ethanol, 1-propanol or isopropanol.

5. Procedure as described in claim 1, wherein the first step the washing can be made from room to the boiling temperature of the solvent and up to 24 hours but, preferably, at the boiling temperature during 6 hours.

6. Washing of the residue produced in the first step of the procedure described in claim 1 with an aliphatic alcohol wherein the number of contaminants is reduced and a material ready for Friedelin extraction is produced.

7. Procedure as described in claim 6 where the preferred aliphatic alcohols are methanol, ethanol, 1-propanol or isopropanol.

8. Procedure as described in claim 1, wherein the extraction of Friedelin during the second step, using preferably an apolar solvent from room temperature to the boiling temperature of the solvent and time periods up to 24 hours, yields a Friedelin-rich extract.

9. Procedure as described in claim 8 where the preferred solvents are chloroform, methylene chloride or diethyl ether.

10. Solvent evaporation of the extract described in claim 8 wherein a Friedelin-rich solid extract is yielded.

11. Procedure as described in claim 1, wherein the third step, a Friedelin sample can be obtained by (re)crystallization of the material obtained in claims 1 and 8, using halogenated solvents, aliphatic hydrocarbons, aliphatic alcohols, ketones, ethers, esters, carboxylic acids and/or acetates as isolated solvents or mixtures.

12. Procedure as described in claim 11 where the preferred solvent mixture is n-hexane: ethyl acetate.

13. Procedure as described in claim 11 wherein the addition of activated charcoal yields a Friedelin material with a reduced concentration of coloured contaminants, as for example suberin precursors.

14. Procedure of re-crystallization of the materials described in claims 11 and 13 with the solvents described in claims 11 and 12 wherein higher purity Friedelin is yielded.

15. Procedure as described in claim 14 wherein the addition of activated charcoal yields a Friedelin material with a reduced concentration of contaminants.

16. Procedure of solubilizing the materials obtained in the procedures described in claims 11, 13, 14 and 15 wherein the solubilisation of these materials in a suitable hot solvent (isolated or as mixtures) with the addition of activated charcoal, yields higher purity Friedelin, after filtration and evaporation of the solvent.

17. Procedure as described in claim 16 where the preferred solvents are n-hexane, chloroform or methylene chloride.

18. Procedure of washing the materials obtained in the procedures described in claims 11, 13, 14, 15 and 16, if necessary, wherein the washing is made with an aliphatic alcohol to separate contaminant remains.

19. Procedure as described in claim 18 where the preferred solvents are methanol, ethanol, 1-propanol or isopropanol.
